# EUROPEAN PATENT APPLICATION

(11) **EP 4 104 771 A1**
(43) Date of publication of application: **21.12.2022**
(21) Application number: 20918934.9
(22) Date of filing: 10.02.2020
(51) Int. Cl.: A61B 17/00, A61F 2/38

(54) **PROSTHESIS**

(71) Applicant: Ames Medical Prosthetic Solutions, S.A.U., 08980 St. Feliu de Llobregat Barcelona (ES); Traumavet S.C.P., 08980 Sant Just Desvern Barcelona (ES)
(72) Inventor: CALERO MARTINEZ, Jose Antonio, 08980 St. Feliu de Llobregat (Barcelona) (ES); MORERA ROCA, Sara, 08980 St. Feliu de Llobregat (Barcelona) (ES); TARRATS GALLOFRE, Emili, 08980 St. Feliu de Llobregat (Barcelona) (ES); TOTUSAUS CALVE, Francisco Javier, 08980 Sant Just Desvern (Barcelona) (ES); LÓPEZ NÁJERA, Diego, 08980 Sant Just Desvern (Barcelona) (ES)
(74) Representative: Herrero & Asociados, S.L.
(86) International application number: PCT/ES2020/070090
(87) International publication number: WO 2021/160901

(57) **Abstract**

The prosthesis comprises a plate (1) and a spacer (2), wherein the plate (1) and the spacer (2) are connected to each other in a tilting manner. Said plate (1) and said spacer (2) are connected by means of a first fastening element (3) defining a tilting axis and a second fastening element (4) movable inside an elongated hole (12) of said plate (1).

It allows to provide a prosthesis, which can be used, in particular, in the tibial tuberosity advancement technique, which is ductile to adapt to the anatomy of the bone, and which is easy to implant and deliver.

## Description

The present invention refers to a prosthesis, in particular, a prosthesis for animals.

### Background of the invention

One of the most frequent injuries or pathologies in canine traumatology is the rupture of the anterior cruciate ligament, which causes degenerative osteoarthritis of the knee joint.

The anterior cruciate ligament undergoes a degenerative process that causes partial and eventually complete rupture. The reason for this pathology is not clear, it could be caused by different factors such as age, overweight, illness, excessive inclination of the tibial plateau, etc.

There are different solutions depending on the type of treatment, which can be conservative or surgical.

As a surgical solution, there are several techniques such as tibial plateau tilt or tibial tuberosity advancement, among others. In both cases, there will be a shift of shear forces from the anterior cruciate ligament to the posterior cruciate ligament, making it the main stabilizer of the knee.

The tibial tuberosity advancement technique consists of performing a medial approach osteotomy on the tibial tuberosity at the level of Gerdi's tubercle and longitudinally on the tibial crest. Once the osteotomy has been performed, the bony fragment of the tibial tuberosity is displaced cranially with the aid of a distractor. The aim of the displacement is to achieve a right angle (90°) between the tibial plateau and the patellar tendon.

This ensures that there is no shear component in the total joint force and no stress on the cruciate ligaments. Finally, a wedge (usually made of titanium) is inserted as a spacer to maintain the separation of the introduced displacement.

Some veterinarians have found that current prostheses do not offer sufficient stability to the assembly, so they use cerclage with stainless steel wire, which improves the fixation and stability of the prosthesis, reinforcing the vertical forces.

However, this technique is complicated to perform, as it is performed in the weakest area of the tibial tuberosity fragment, where the bone thickness is the smallest, in order to reinforce it. Depending on the twisting force exerted by screwing in the wire, the result may be a cerclage that is too strong (the wire breaks) or too loose (it does not perform its function).

### Description of the invention

Therefore, an objective of the present invention is to provide a prosthesis, which can be used in particular in the technique of tibial tuberosity advancement, which is ductile to adapt to the anatomy of the bone, and which is easy to implant and deliver.

The prosthesis of the invention solves the aforementioned disadvantages and has other advantages that will be described below.

The prosthesis according to the present invention comprises a plate and a spacer, wherein the plate and the spacer are connected to each other in a tilting manner.

According to a preferred embodiment, said plate and said spacer are connected by means of a first fastening element defining a tilting axis and a second fastening element movable inside an elongated hole in said plate.

Advantageously, the plate comprises a junction area, where the spacer is positioned, and an arm, and the first and second fastening elements are arranged at two opposite ends of the junction area.

Furthermore, said arm preferably comprises an extension provided with a hole, which preferably is substantially perpendicular with respect to the longitudinal axis of said arm.

Advantageously, said arm comprises a hole at its end furthest from said junction area, and said junction area comprises a plurality of elongated holes.

According to a preferred embodiment, the spacer comprises a central hole, and the spacer is of progressively decreasing thickness and comprises a curved surface.

The prosthesis according to the present invention has, among others, the following advantages:
- It is very simple, offering the least possible aggression to the patient.
- It is simple for the surgeon.
- It has clinical effectiveness in relation to biocompatibility, cytotoxicity, osseointegration, etc.
- It has a reduced cost.
- It facilitates its delivery, as it does not need to be delivered sterile and can be sterilized in the clinics.

### Brief description of the drawings

For a better understanding of what has been explained above, some drawings are included in which, schematically and only by way of a non-limiting example, a practical case of embodiment is shown.
Figure 1 is an exploded perspective view of the prosthesis according to the present invention; and

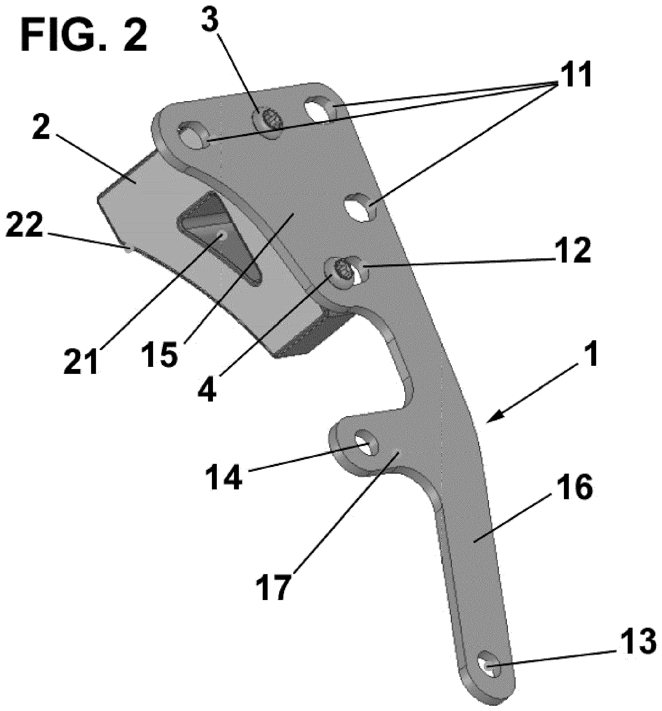

Figure 2 is a perspective view of the prosthesis according to the present invention.

### Description of a preferred embodiment

As shown in the figures, the prosthesis according to the present invention comprises a plate, indicated in general by reference number 1, and a spacer 2, which is mounted on said plate 1 in a tilting manner.

The connection between the plate 1 and the spacer 2 is made by means of two fastening elements, a first fastening element 3, which defines a tilting axis and allows the plate 1 to tilt with respect to the spacer 2, and a second fastening element 4, which is housed in an elongated hole 12 of the plate 1, so that it can be placed in any position inside said elongated hole 12.

These first and second fastening elements 4 are, for example, screws.

This elongated hole 12 allows the surgeon to find the medial distal fixation by tilting the plate 1 relative to the spacer 2.

The plate 1, which preferably is made of grade 2 titanium, comprises a junction area 15 and an arm 16, the contour of the junction area 15 substantially corresponding to the profile of the spacer 2, and said junction area 15 comprising a plurality of holes 11, which preferably are also elongated in order to be able to incline fixing screws (not shown) to find the suitable bony zone.

Preferably, two of said holes 11 are aligned with the first fastening element 3.

As can be seen in the figures, the first fastening element 3 is arranged at one end of the junction area 15 and the second fastening element 4 is arranged at the other end of the junction area 15.

The arm 16 of the plate 1 also preferably comprises an extension 17, which is substantially perpendicular with respect to the longitudinal axis of the arm 16, and which comprises a hole 14 therein. This extension 17 allows for improved fixation and stabilization of the prosthesis.

In addition, the end of the arm 16 furthest from the junction area 15 comprises a distal anchor hole 13, the position of which relative to the spacer 2 changes depending on the rotation of the plate 1.

The spacer 2 is made of a biocompatible material and has a central hole 21 to promote osseointegration and the incorporation of bone cement.

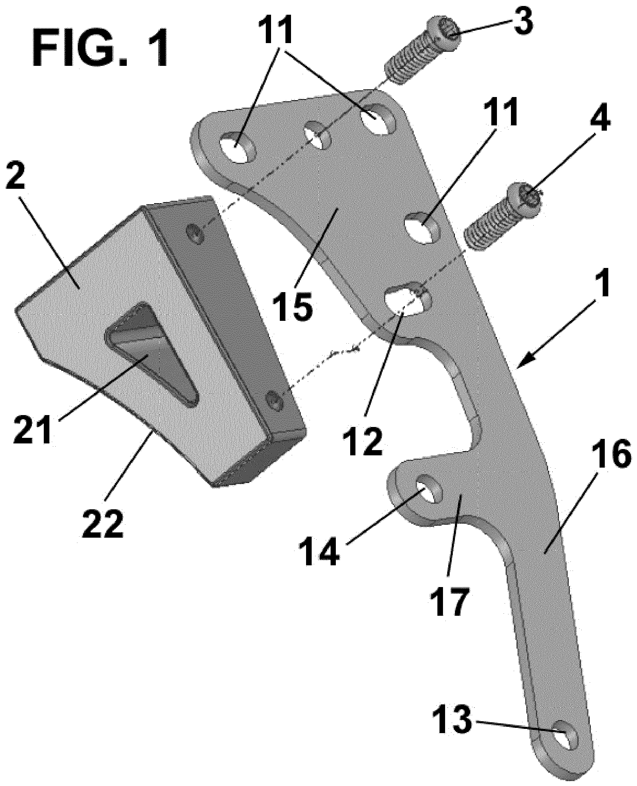

In addition, it has a geometry, which includes a curved surface 22, that mimics the contralateral tibia, preventing it from protruding. This geometry can be seen in greater detail in figure 1, and its thickness is decreasing, i.e., its thickness changes along its length.

The use of the prosthesis according to the present invention is very similar to currently known prostheses for the tibial tuberosity advancement technique, with the advantages indicated above.

Although reference has been made to a specific embodiment of the invention, it is obvious to a person skilled in the art that the prosthesis described is susceptible to numerous variations and modifications, and that all the details mentioned can be replaced by technically equivalent ones, without departing from the scope of protection defined by the appended claims.

## Claims

1. Prostheses, comprising:
- a plate (1); and
- a separator (2),
**characterized in that** the plate (1) and the spacer (2) are connected to each other in a tilting manner.

2. Prosthesis according to claim 1, wherein said plate (1) and said spacer (2) are connected by means of a first fastening element (3) defining a tilting axis and a second fastening element (4) movable inside an elongated hole (12) of said plate (1).

3. Prosthesis according to claim 1, wherein the plate (1) comprises a junction area (15), where the spacer (2) is placed, and an arm (16).

4. Prosthesis according to claims 2 and 3, wherein the first and second fastening elements (3, 4) are arranged at two opposite ends of said junction area (15).

5. Prosthesis according to claim 3, wherein said arm (16) comprises an extension (17) provided with a hole (14).

6. Prosthesis according to claim 5, wherein said extension (17) is substantially perpendicular with respect to the longitudinal axis of said arm (16).

7. Prosthesis according to claim 3, wherein said arm (16) comprises a hole (13) at its end furthest from said junction area (15).

8. Prosthesis according to claim 3, wherein said junction area (15) comprises a plurality of elongated holes (11).

9. Prosthesis according to claim 1, wherein the spacer (2) comprises a central hole (21).

10. Prosthesis according to claim 1, wherein the spacer (2) is of progressively decreasing thickness.

11. Prosthesis according to claim 1, wherein the spacer (2) comprises a curved surface (22).
